# EUROPÄISCHE PATENTANMELDUNG

(11) **EP 1 797 865 A2**
(43) Veröffentlichungstag der Anmeldung: **20.06.2007**
(21) Anmeldenummer: 06020916.0
(22) Anmeldetag: 05.10.2006
(51) Int. Cl.: A61K 8/44, A61K 8/46, A61Q 5/06, A61Q 5/10

(54) **Verfahren zum gleichzeitigen Färben und Verformen keratinhaltiger Fasern**

(30) Priorität: 19.12.2005 DE 102005061023
(71) Anmelder: HENKEL KOMMANDITGESELLSCHAFT AUF AKTIEN, 40589 Düsseldorf (DE)
(72) Erfinder: Müller, Burkhard, 21075 Hamburg (DE); Knappe, Thorsten, 22869 Schenefeld (DE)

(57) **Zusammenfassung**

Verfahren zum gleichzeitigen Färben und dauerhaften Verformen keratinhaltiger Fasern, insbesondere menschlicher Haare, wobei (i) eine wässrige, keratinreduzierende Zusammensetzung, enthaltend mindestens eine keratinreduzierende Verbindung und mindestens ein Oxidationsfarbstoffvorprodukt und/oder mindestens einen direktziehenden Farbstoff, auf die Fasern aufgetragen wird, (ii) die Fasern nach einer Einwirkzeit Z1 gespült und gegebenenfalls getrocknet werden, (iii) die Fasern anschließend unter Zuhilfenahme von Verformungshilfsmitteln verformt werden, wobei die Fasern vorzugsweise zwischen den Schritten (ii) und (iii) keinerlei weitere Behandlung erfahren, und (iv) schließlich eine oxidierende Zusammensetzung, enthaltend mindestens eine oxidierende Verbindung, auf die Fasern aufgetragen und nach einer Einwirkzeit Z2 wieder abgespült wird.

## Beschreibung

Die Erfindung betrifft ein Verfahren zum gleichzeitigen Färben und dauerhaften Verformen keratinhaltiger Fasern, insbesondere menschlicher Haare.

Als keratinhaltige Fasern können prinzipiell alle tierischen Haare, z.B. Wolle, Rosshaar, Angorahaar, Pelze, Federn und daraus gefertigte Produkte oder Textilien eingesetzt werden. Bevorzugt wird die Erfindung jedoch im Rahmen einer Haarumformung, insbesondere der Dauerwellung glatter oder der Glättung krauser menschlicher Haare und daraus gefertigter Perücken, eingesetzt.

Eine dauerhafte Verformung keratinhaltiger Fasern wird üblicherweise derart durchgeführt, dass man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann in dem sogenannten Fixierschritt mit einer Oxidationsmittelzubereitung behandelt, gespült und nach oder während des Fixierschritts von den Verformungshilfsmitteln (Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R-S-S-R + HSO₃⁽⁻⁾ → R-SH + R-S-SO₃⁽⁻⁾

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glattgekämmt. Anstelle des Wicklers ist es auch möglich, die Faser auf ein Glättungsboard glattzulegen. Glättungsboarde sind üblicherweise rechteckige Tafeln z.B. aus Kunststoff.

Wenn zusätzlich zur Umformung auch eine Färbung der keratinischen Faser gewünscht wird, kann die Färbung als separate Behandlung vor oder nach der erfolgten Umformung durchgeführt werden. Insbesondere im Falle einer oxidativen Färbung führt dies aber zu einer extremen Beanspruchung der Faser, da jede oxidative Behandlung der Faser deren innere Struktur schädigt. Zudem ist ein solches Vorgehen sehr zeitaufwändig.

Es wurden daher bereits einige Verfahren zur gleichzeitigen Umformung und Färbung keratinischer Fasern, insbesondere Haare, vorgeschlagen. In vielen Fällen wird dazu im Fixierschritt eine Oxidationsmittelzubereitung eingesetzt, die neben dem Oxidationsmittel einen direktziehenden Farbstoff und/oder ein Oxidationsfarbstoffvorprodukt enthält. Ein entsprechendes Vorgehen ist beispielsweise in DE 197 13 698 C1 beschrieben. Dieses Vorgehen hat jedoch den Nachteil, dass die Färbung gleichzeitig mit der Fixierung, d.h. zu einem Zeitpunkt erfolgt, zu dem die zu behandelnden Fasern auf ein Verformungshilfsmittel aufgebracht sind und dadurch unter mechanischer Spannung stehen. Dies behindert das gleichmäßige Aufbringen der Farbstoffe, so dass die Gefahr eines ungleichmäßigen Färbeergebnisses besteht.

Aus EP 0 352 375 A1 und EP 1 287 812 A2 sind Verfahren zur gleichzeitigen Umformung und Färbung von Haaren bekannt, bei denen eine keratinreduzierende Zubereitung eingesetzt wird, die bereits die notwendigen Farbstoffe und/oder Farbstoffvorprodukte enthält. Zumindest ein Teil der jeweiligen keratinreduzierenden Zubereitung wird auf das Haar aufgebracht, nachdem dieses mechanisch verformt wurde. Es ergibt sich daher wiederum das Problem, dass die Farbstoffe nicht völlig gleichmäßig auf dem Haar verteilt werden können, und daher das Färbeergebnis ungleichmäßig ausfällt. Zudem ist das Umformungsergebnis hinsichtlich des Umformungsgrades und der Gleichmäßigkeit der Umformung verbesserungswürdig.

Aufgabe der Erfindung ist es daher, ein Umformungsverfahren für keratinhaltige Fasern, insbesondere für menschliches Haar, bereitzustellen, das ein verbessertes Umformungsergebnis liefert, wobei die Faser gleichzeitig gleichmäßig gefärbt wird.

Überraschenderweise wurde gefunden, dass die Aufgabe durch ein Verfahren gelöst wird, bei dem die keratinhaltigen Fasern, insbesondere menschlichen Haare, zunächst mit einer färbenden Reduktionsmittelzubereitung behandelt, dann gespült und erst nachdem die Reduktionsmittelzubereitung ausgespült wurde, unter Zuhilfenahme von Verformungshilfsmitteln verformt werden.

Ein erster Gegenstand der Erfindung ist daher ein Verfahren zum gleichzeitigen Färben und dauerhaften Verformen keratinhaltiger Fasern, insbesondere menschlicher Haare, wobei
(i) eine wässrige, keratinreduzierende Zusammensetzung, enthaltend mindestens eine keratinreduzierende Verbindung und mindestens ein Oxidationsfarbstoffvorprodukt und/oder mindestens einen direktziehenden Farbstoff, auf die Fasern aufgetragen wird,
(ii) die Fasern nach einer Einwirkzeit Z1 gespült und gegebenenfalls getrocknet werden,
(iii) die Fasern anschließend unter Zuhilfenahme von Verformungshilfsmitteln verformt werden und
(iv) schließlich eine oxidierende Zusammensetzung, enthaltend mindestens eine oxidierende Verbindung, auf die Fasern aufgetragen und nach einer Einwirkzeit Z2 wieder abgespült wird.

Das erfindungsgemäße Verfahren zeichnet sich insbesondere durch eine besondere Gleichmäßigkeit der Umformung, sowie ein gleichmäßiges und intensives Färbeergebnis aus.

Verformungshilfsmittel im Sinne des erfindungsgemäßen Verfahrens können
- z.B. Lockenwickler oder Papilloten im Falle einer Dauerwelle,
- oder Hilfsmittel für eine mechanische Glättung, wie ein Kamm oder eine Bürste, ein Glättungsboard oder ein beheizbares Glättungseisen im Falle einer Haarglättung sein.

Wenn die Verformungshilfsmittel, beispielsweise Wickler, im Rahmen eines Dauerwellverfahrens für einen längeren Zeitraum an der Faser befestigt werden, so kann es zweckmäßig sein, diese Verformungshilfsmittel vor, während oder nach Schritt (iv) zu entfernen. Es kann in diesem Zusammenhang vorteilhaft sein, die Verformungshilfsmittel während des Schritts (iv) im Haar zu belassen, sie danach zu entfernen und danach Schritt (iv) als sogenannten Nachfixierschritt (v) zu wiederholen.

Ein entscheidender Schritt des erfindungsgemäßen Verfahrens ist das Ausspülen der Reduktionsmittelzubereitung vor der mechanischen Verformung der Fasern. Dadurch wird gewährleistet, dass die Farbstoffe auch während der mechanischen Verformung und der Fixierung der Fasern gleichmäßig über die gesamte Faser verteilt sind.

In einer bevorzugten Ausführungsform des erfindungsgemäßen Verfahrens erfahren die keratinischen Fasern zwischen den Behandlungsschritten (ii) und (iii) keinerlei weitere Behandlung.

In einer weiteren bevorzugten Ausführungsform der Erfindung werden die keratinhaltigen Fasern vor dem Schritt (i) angefeuchtet. Dies kann durch Besprühen der Fasern mit einer Flüssigkeit, bevorzugt mit Wasser, geschehen. Bevorzugterweise werden die Fasern vor Schritt (i) mit einem herkömmlichen Shampoo shampooniert, gespült und dann mit einem Handtuch frottiert. Nach Abschluß des Frottierschritts bleibt eine fühlbare Restfeuchtigkeit im Haar zurück.

Die Einwirkzeit Z1 beträgt bevorzugt 5-60 Minuten, besonders bevorzugt 10-30 Minuten. Die Einwirkzeit Z2 beträgt bevorzugt 1-30 Minuten, besonders bevorzugt 5-20 Minuten.

Eine wässrige Zusammensetzung im Sinne der Erfindung enthält mindestens 50 Gew.% Wasser bezogen auf das Gewicht der gesamten Zusammensetzung. Diese wässrige Zusammensetzung kann in verschiedenen Formen, beispielsweise als Lotion, Öl-in-Wasser-Emulsion oder Wasser-in-Öl-Emulsion, vorliegen.

Die in der wässrigen Zusammensetzung des Schritts (i) enthaltenen keratinreduzierenden Verbindungen werden bevorzugt ausgewählt aus Verbindungen mit mindestens einer Thiolgruppe sowie deren Derivate, aus Sulfiten, Hydrogensulfiten und Disulfiten.

Verbindungen mit mindestens einer Thiolgruppe sowie deren Derivate sind beispielsweise Thioglykolsäure, Thiomilchsäure, Thioäpfelsäure, Phenylthioglykolsäure, Mercaptoethansulfonsäure sowie deren Salze und Ester (wie z.B. Isooctylthioglycolat und Isopropylthioglycolat), Cysteamin, Cystein, Bunte Salze und Salze der schwefligen Säure. Bevorzugt geeignet sind die Monoethanolammoniumsalze- oder Ammoniumsalze der Thioglykolsäure und/oder der Thiomilchsäure sowie die freien Säuren. Diese werden in der wässrigen Zusammensetzung bevorzugt in Konzentrationen von 0,5 bis 2,0 mol/kg bei einem pH-Wert von 5 bis 12, insbesondere von 7 bis 9,5, eingesetzt. Zur Einstellung dieses pH-Wertes enthalten die wässrigen Zusammensetzungen üblicherweise Alkalisierungsmittel wie Ammoniak, Alkali- und Ammonium-carbonate und - hydrogencarbonate oder organische Amine wie Monoethanolamin.

Beispiele für keratinreduzierende Verbindungen der Disulfite, die in der wässrigen Zusammensetzung enthalten sein können, sind Alkalidisulfite, wie z.B. Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für keratinreduzierende Verbindungen der Hydrogensulfite, die in der wässrigen Zusammensetzung enthalten sein können, sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Verbindungen der Sulfite, die in der wässrigen Zusammensetzung enthalten sein können, sind Sulfite als Alkali-, Ammonium- oder Alkanolammonium-Salz auf Basis eines C₂-C₄-Mono-, Di- oder Trialkanolamins. Ammoniumsulfit ist dabei bevorzugt. Der pH-Wert der wässrigen Zusammensetzung wird bei Verwendung von Sulfit und/oder Disulfit und/oder Hydrogensulft bevorzugt auf einen Wert im Neutralbereich von pH 5 bis 8, bevorzugt von pH 6 bis 7,5 eingestellt.

Bevorzugte C₂-C₄-Alkanolamine sind erfindungsgemäß 2-Aminoethanol (Monoethanolamin) und N,N,N-Tris(2-hydroxyethyl)amin (Triethanolamin). Monoethanolamin ist ein besonders bevorzugtes C₂-C₄-Alkanolamin, das insbesondere in einer Menge von 0,2 bis 6 Gew.-% bezogen auf die gesamte wässrige Zusammensetzung, eingesetzt wird.

Die in der wässrigen Zusammensetzung des Schritts (i) enthaltenen keratinreduzierenden Verbindungen werden besonders bevorzugt ausgewählt aus Thioglykolsäure, Thiomilchsäure und Cystein sowie deren Salze.

Die keratinreduzierende Verbindung wird bevorzugt in einer Menge von 1 bis 25 Gew.-%, besonders bevorzugt in einer Menge von 5 bis 15 Gew.-%, bezogen auf die gesamte wässrige, keratinreduzierende Zusammensetzung, eingesetzt.

Die in Schritt (i) des erfindungsgemäßen Verfahrens eingesetzte wässrige, keratinreduzierende Zusammensetzung enthält neben der keratinreduzierenden Verbindung mindestens ein Oxidationsfarbstoffvorprodukt und/oder mindestens einen direktziehenden Farbstoff.

Oxidationsfarbstoffvorprodukte lassen sich unterteilen in sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Eine oxidative Färbung erfordert daher immer den Einsatz wenigstens einer Entwicklerkomponente. Direktziehende Farbstoffe werden auch als Direktzieher bezeichnet. Es handelt sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 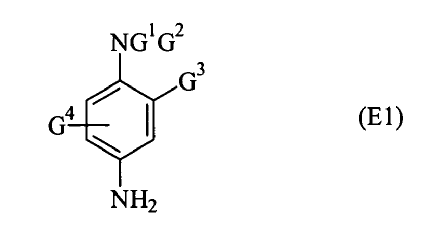 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Jod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den Entwicklerkomponenten genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin, N-(4-Amino-3-methylphenyl)-N-[3-(1 H-imidazol-1-yl)propyl]amin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 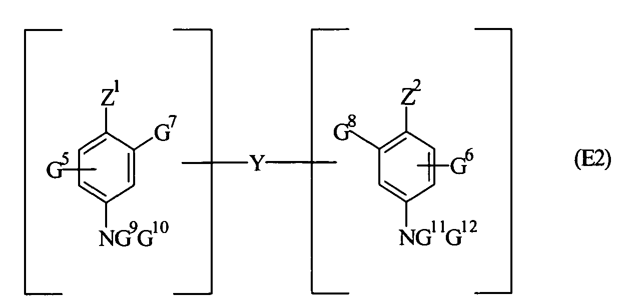 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest, mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-((3-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Bis-(2-hydroxy-5-aminophenyl)-methan ist eine ganz besonders bevorzugte zweikernige Entwicklerkomponente der Formel (E2).

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 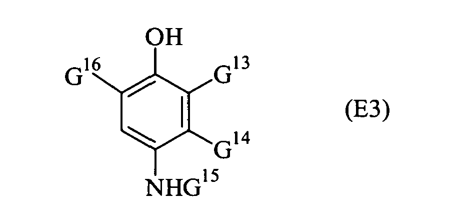 wobei:
- G¹³ steht für ein Wasserstoffatom, ein Halogenatom, einen C₁- bis C₄-Alkylrest, einen C₁-bis C₄-Monohydroxyalkylrest, einen C₂- bis C₄-Polyhydroxyalkylrest, einen (C₁- bis C₄)-Alkoxy-(C₁-bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 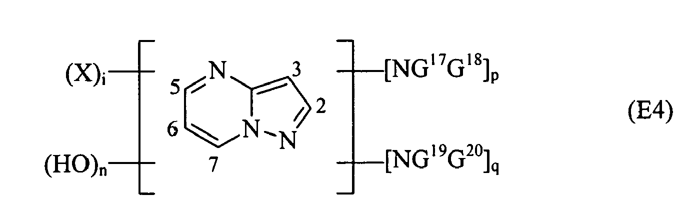 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄- Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

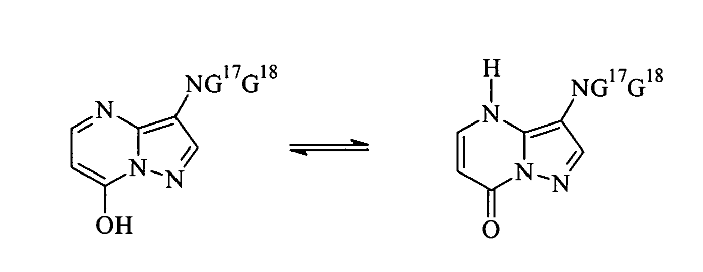

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Als Kupplerkomponenten können beispielsweise m-Phenylendiaminderivate, Naphthole, Resorcin und Resorcinderivate, Pyrazolone und m-Aminophenolderivate verwendet werden. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 2-({3-[(2-Hydroxyethyl)amino]-4-methoxy-5-methylphenyl}amino)ethanol, 2-({3-((2-Hydroxyethyl)amino]-2-methoxy-5-methylphenyl}amino)ethanol, 2-({3-[(2-Hydroxyethyl)amino]-4,5-dimethylphenyl}amino)ethanol und 2-[(3-Morpholin-4-ylphenyl)amino]ethanol.

Sowohl die Entwicklerkomponenten als auch die Kupplerkomponenten werden bevorzugt in einer Menge von 0,005 bis 20 Gew.-%, vorzugsweise 0,1 bis 5 Gew.-%, jeweils bezogen auf die gesamte wässrige, keratinreduzierende Zusammensetzung, zugegeben. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

Geeignete direktziehende Farbstoffe sind beispielsweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen, sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können kationische direktziehende Farbstoffe eingesetzt werden. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quartäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

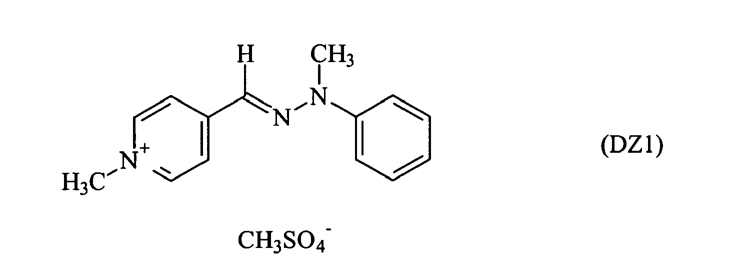

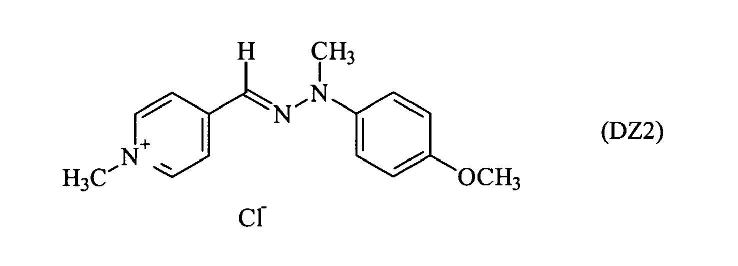

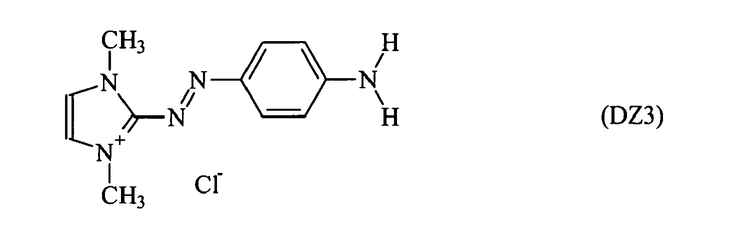

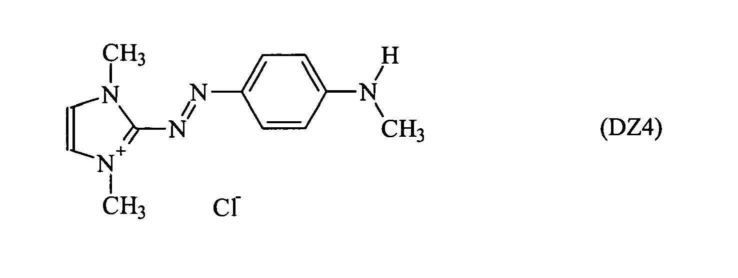

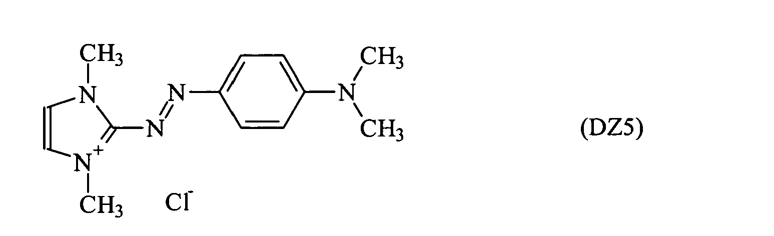

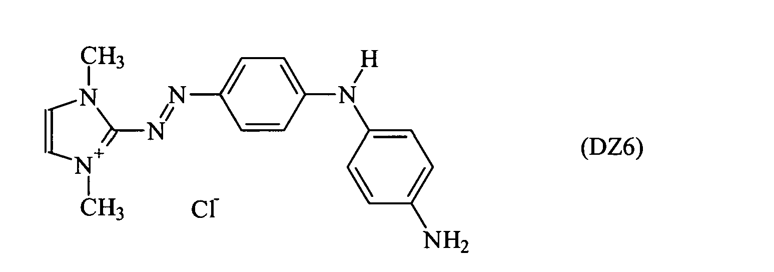

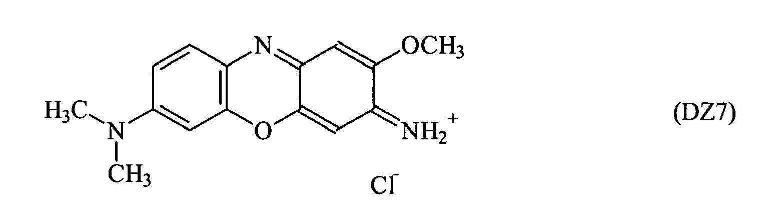

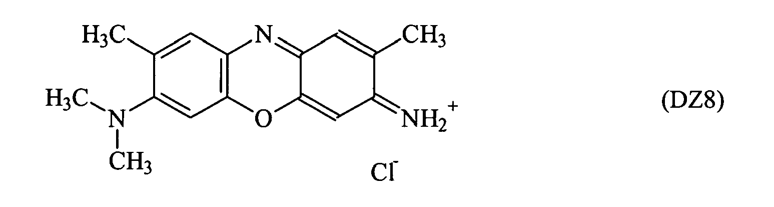

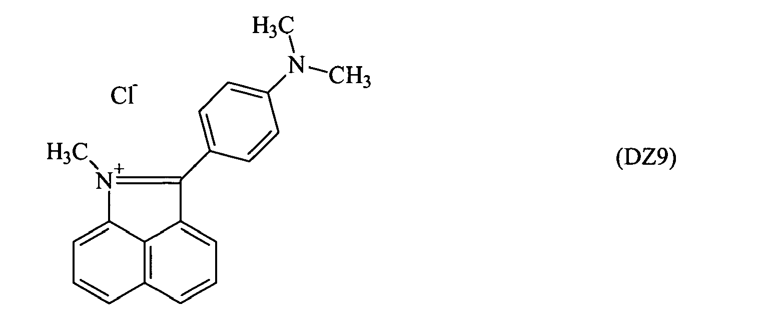

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Weiterhin können auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, zugemischt werden.

Die direktziehenden Farbstoffe werden bevorzugt in einer solchen Menge zugegeben, dass die Gesamtmenge an direktziehendem Farbstoff 0,1 bis 20 Gew.-%, vorzugsweise 0,1 bis 5 Gew.-%, jeweils bezogen auf die wässrige, keratinreduzierende Zusammensetzung, beträgt.

Neben den genannten Farbstoff-Komponenten können der wässrigen, keratinreduzierenden Zusammensetzung mindestens eine Vorstufe eines naturanalogen Farbstoffs zugemischt werden. Als Vorstufen naturanaloger Farbstoffe werden bevorzugt Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren bevorzugten Ausführungsform werden die Verbindungen der Formel (I) im Gemisch mit mindestens einem Indol- und/oder Indolinderivat eingesetzt.

Besonders gut als Vorstufen naturanaloger Haarfarbstoffe geeignet sind Derivate des 5,6-Dihydroxyindolins der Formel (NAV I), 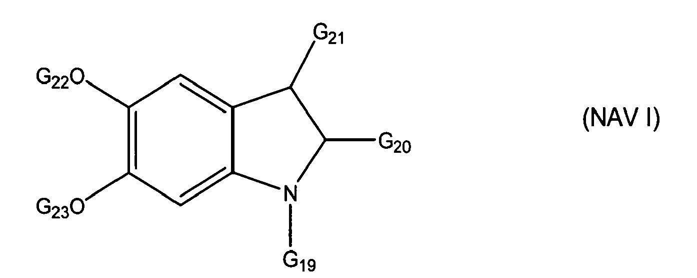 in der unabhängig voneinander
- G₁₉ steht für Wasserstoff, eine C₁-C₄-Alkylgruppe oder eine C₁-C₄-Hydroxyalkylgruppe,
- G₂₀ steht für Wasserstoff oder eine -COOH-Gruppe, wobei die -COOH-Gruppe auch als Salz mit einem physiologisch verträglichen Kation vorliegen kann,
- G₂₁ steht für Wasserstoff oder eine C₁-C₄-Alkylgruppe,
- G₂₂ steht für Wasserstoff, eine C₁-C₄-Alkylgruppe oder eine Gruppe -CO-G₂₄, in der G₂₄ steht für eine C₁-C₄-Alkylgruppe, und
- G₂₃ steht für eine der unter G₂₂ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (NAV II), 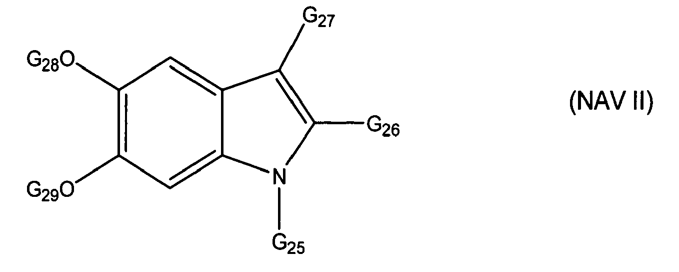 in der unabhängig voneinander
- G₂₅ steht für Wasserstoff, eine C₁-C₄-Alkylgruppe oder eine C₁-C₄-Hydroxyalkylgruppe,
- G₂₆ steht für Wasserstoff oder eine -COOH-Gruppe, wobei die -COOH-Gruppe auch als Salz mit einem physiologisch verträglichen Kation vorliegen kann,
- G₂₇ steht für Wasserstoff oder eine C₁-C₄-Alkylgruppe,
- G₂₈ steht für Wasserstoff, eine C₁-C₄-Alkylgruppe oder eine Gruppe -CO-G₃₀, in der G₃₀ steht für eine C₁-C₄-Alkylgruppe, und
- G₂₉ steht für eine der unter G₂₈ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate werden üblicherweise in Mengen von 0,05-10 Gew.-%, vorzugsweise 0,2-5 Gew.-% eingesetzt.

In einer weiteren Ausführungsform kann es erfindungsgemäß bevorzugt sein, das Indolin- oder Indolderivat in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Es ist nicht erforderlich, dass die genannten Farbstoffe oder Farbstoffvorprodukte jeweils einheitliche Verbindungen darstellen. Vielmehr können in den wässrigen, keratinreduzierenden Zusammensetzungen, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die Gesamtmenge an Oxidationsfarbstoffvorprodukten und/oder direktziehenden Farbstoffen in der wässrigen, keratinreduzierenden Zusammensetzung beträgt vorzugsweise 0,1 bis 20 Gew.-%, bezogen auf die gesamte wässrige, keratinreduzierende Zusammensetzung.

Darüberhinaus kann die wässrige, keratinreduzierende Zusammensetzung weitere Komponenten enthalten, die die Wirkung der keratinreduzierenden Verbindung auf das Keratin fördern. Solche Komponenten sind z.B. Quellmittel für keratinhaltige Fasern wie z.B. C₁-C₆-Alkohole und wasserlösliche Glykole oder Polyole wie z.B. Glycerin, 1,2-Propylenglykol oder Sorbit und Harnstoff oder Harnstoffderivate wie z.B. Allantoin und Guanidin sowie Imidazol und dessen Derivate. In einer bevorzugten Ausführung enthält die wässrige Zusammensetzung 0,05 bis 5 Gew.-% 1,2-Propylenglykol und/oder 0,05 bis 5 Gew.-% Harnstoff. Die Mengenangaben beziehen sich jeweils auf die gesamte wässrige Zusammensetzung.

In einer besonderen Ausführungsform wird die wässrige, keratinreduzierende Zusammensetzung, die in Schritt (i) des erfindungsgemäßen Verfahrens zum Einsatz kommt, durch Mischen einer wässrigen Zusammensetzung, enthaltend mindestens eine keratinreduzierende Verbindung, und einer weiteren wässrigen Zusammensetzung, enthaltend mindestens ein Oxidationsfarbstoffvorprodukt und/oder mindestens einen direktziehenden Farbstoff, erhalten.

Vorzugsweise erfolgt das Mischen unmittelbar vor dem Aufbringen der Mischung auf die zu behandelnden keratinhaltigen Fasern. Dieses Vorgehen hat insbesondere den Vorteil, dass Stabilitätsprobleme bezüglich der eingesetzten Inhaltsstoffe sowie der fertigen Zusammensetzung, die sich beim Einarbeiten der Farbstoffe und/oder Farbstoffvorprodukte in eine Reduktionsmittelhaltige Zusammensetzung ergeben können, vermieden werden. Die benötigten wässrigen Zusammensetzungen, d.h. die wässrige Zusammensetzung, enthaltend mindestens eine keratinreduzierende Verbindung, die wässrige Zusammensetzung, enthaltend mindestens ein Oxidationsfarbstoffvorprodukt und/oder mindestens einen direktziehenden Farbstoff, sowie die oxidierende Zusammensetzung, die zur Durchführung von Schritt (iv) des erfindungsgemäßen Verfahrens benötigt wird, können jeweils in einen separaten Behälter verpackt und gemeinsam als Kit, enthaltend diese Behälter, angeboten werden.

Eine trockene keratinhaltige Faser gemäß Schritt (ii) des erfindungsgemäßen Verfahrens liegt dann vor, wenn die den Haaren anhaftenden Wasserreste soweit verdampft sind, dass die Haare einzeln fallen. Bevorzugt ist bei einer trockenen keratinhaltigen Faser entweder der Feuchtigkeitsgehalt der Faser mit der Feuchtigkeit der Luft im wesentlichen im Gleichgewicht oder die Faser nimmt Feuchtigkeit aus der Luft der Umgebung auf.

In Schritt (iv) des erfindungsgemäßen Verfahrens werden die verformten keratinischen Fasern durch Aufbringen einer oxidierenden Zusammensetzung fixiert. Gleichzeitig gewährleistet das Aufbringen eines Oxidationsmittels, dass gegebenenfalls noch nicht abreagierte Oxidationsfarbstoffvorprodukte zu den entsprechenden Farbstoffen umgesetzt werden. Die in der oxidierenden Zusammensetzung enthaltene oxidierende Verbindung besitzt ein solches RedoxPotential, dass zwei Mercaptogruppen unter Bildung einer Disulfidbrücke oxidiert werden können. Ein bevorzugtes Oxidationsmittel wird ausgewählt aus z.B. Natriumbromat, Kaliumbromat oder Wasserstoffperoxid. Es ist besonders bevorzugt, Wasserstoffperoxid als Oxidationsmittel zu verwenden. Zur Stabilisierung wässriger Wasserstoffperoxidzubereitungen können zusätzlich übliche Stabilisatoren zugesetzt werden. Der pH-Wert der wässrigen H₂O₂-Zubereitungen, die anwendungsfertig üblicherweise etwa 0,5 bis 3,0 Gew.-% H₂O₂ enthalten, liegt bevorzugt bei 2 bis 6. Es kann auch von Konzentraten mit üblicherweise bis zu 30 Gew.-% H₂O₂ ausgegangen werden, die vor der Anwendung entsprechend verdünnt werden. Hierbei kann es bevorzugt sein, handelsübliche Schnellfixierungen, beispielsweise das Produkt Natural Styling Rinse Neutraliser 1:4 der Firma Henkel, einzusetzen. Enthält die oxidierende Zusammensetzung Bromat als Oxidationsmittel, so ist dies üblicherweise in Konzentrationen von 1 bis 10 Gew.-% enthalten und der pH-Wert der Lösungen wird auf 4 bis 7 eingestellt.

Sowohl die wässrige, keratinreduzierende Zusammensetzung, als auch die oxidierende Zusammensetzung können weiterhin alle für solche Zusammensetzungen üblichen, weiteren Inhaltsstoffe enthalten. Solche Inhaltsstoffe können beispielsweise Konditioniermittel, Tenside, Emulgatoren, UV-Stabilisatoren, Konservierungsmittel, Verdicker oder viskositätserhöhende Zusätze sein.

Vorzugsweise enthält die wässrige, keratinreduzierende Zusammensetzung und/oder die oxidierende Zusammensetzung mindestens einen oberflächenaktiven Stoff aus der Gruppe der anionischen, amphoteren, zwitterionischen und nichtionischen Tenside. Die Tenside haben unter anderem die Aufgabe, die Benetzung der Keratinoberfläche durch die Behandlungslösung zu fördern.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 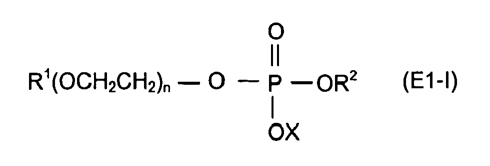 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder
   CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III) 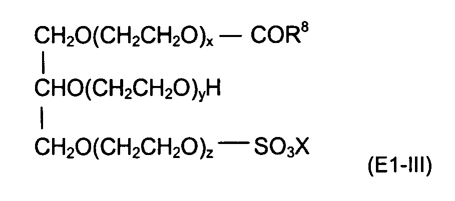 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, wie sie beispielsweise in der EP 0 561 825 B1, der EP 0 561 999 B1, der DE 42 04 700 A1 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind,
- Amidethercarbonsäuren wie sie in der EP 0 690 044 beschrieben sind,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Journal Heft 8, 598 (1995) verwiesen.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 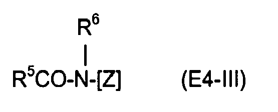 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424, US 2,016,962 und US 2,703,798 sowie die Internationale Patentanmeldung WO 92/06984 verwiesen. Eine Übersicht zu diesem Thema von H.Kelkenberg findet sich in Tens. Surf. Det. 25, 8 (1988). Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die Tenside werden in Mengen von 0,1 bis 45 Gew.%, bevorzugt 0,5 bis 30 Gew.% und ganz besonders bevorzugt von 0,5 bis 25 Gew.%, bezogen auf die jeweilige gesamte erfindungsgemäß verwendete Zusammensetzung, eingesetzt.

In einer weiteren Ausführungsform können in den eingesetzten Zusammensetzungen Emulgatoren verwendet werden. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D. Dörfler, Grenzflächen- und Kolloidchemie, VCH Verlagsgesellschaft mbH. Weinheim, 1994". Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Bevorzugt sind nichtionogene Emulgatoren mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen. Nichtionogene Emulgatoren mit einem HLB-Wert von 10 bis 16 können erfindungsgemäß besonders bevorzugt sein.

Die wässrige, keratinreduzierende Zusammensetzung und/oder die oxidierende Zusammensetzung können weiterhin mindestens einen linearen oder verzweigten, gesättigten oder ungesättigten Fettalkohol enthalten. Als Fettalkohole können eingesetzt werden Fettalkohole mit C₆-C₃₀-, bevorzugt C₁₀-C₂₂- und ganz besonders bevorzugt C₁₂-C₂₂- Gruppen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Fettalkohole werden beispielsweise in Mengen von 0,1 bis 20 Gew.-%, bezogen auf die gesamte Zubereitung, bevorzugt in Mengen von 0,1 bis 10 Gew.-% eingesetzt.

Die wässrige, keratinreduzierende Zusammensetzung und/oder die oxidierende Zusammensetzung können zusätzlich eine viskositätserhöhende Verbindung, im weiteren als Verdickungsmittel bezeichnet, enthalten.

Erfindungsgemäß einsetzbare Verdickungsmittel sind beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol, sowie viskositätserhöhende Polymere auf Polyacrylat-Basis wie sie beispielsweise unter den Handelsnamen Pemulen^{®}, Aculyn^{®} und Carbopol^{®} vertrieben werden. Desweiteren wird bevorzugt eine Mischung aus Diestern von 1,2-Propylenglykol mit Fettsäuren, beispielsweise der Verdicker mit der INCI-Bezeichnung Propylene Glycol Dicaprylate / Dicaprate, in den erfindungsgemäßen Mitteln eingesetzt.

Weiterhin können folgende Verbindungen enthalten sein:
- lineare und/oder verzweigte Fettsäuren, bevorzugt C₂-C₃₀-Fettsäuren, besonders bevorzugt C₄-C₁₈ Fettsäuren, am meisten bevorzugt C₆-C₁₀-Fettsäuren und/oder deren physiologisch verträglichen Salzen; weitere Beispiele sind Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Milchsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure,
- Polyhydroxyverbindungen; hierbei sind insbesondere zu nennen
   - Zucker mit 5 und/oder 6 Kohlenstoffatomen, insbesondere als Mono- und/oder Oligosaccharide, beispielsweise Glucose, Fructose, Galactose, Lactose, Arabinose, Ribose, Xylose, Lyxose, Allose, Altrose, Mannose, Gulose, Idose, Talose und Sucrose und/oder deren Derivate, z.B. Etherderivate, Aminoderivate und/oder Acetylderivate wie acetylierte Glucose, z.B. Tetraacetylglucose, Pentaacetylglucose und/oder 2-Acetamido-2-desoxyglucose. Bevorzugte Zuckerbausteine sind Glucose, Fructose, Galactose, Allose, Lactose, Arabinose und Sucrose; Glucose, Galactose und Lactose sind besonders bevorzugt;
   - Onsäuren, insbesondere Gluconsäure, Glucuronsäure;
   - Polyole, wie z.B. Glucamine, Glycerin, Mono- oder Diglyceride, 2-Ethyl-1,3-hexandiol, 2-Hydroxymethylpropantriol, Glycole wie Ethylenglykol, Diethylenglykol, Triethylenglykol, Propylenglykol, Dipropylenglykol, 1,3-Butandiol;
   - Polyhydroxysäuren, wie z.B. Pentahydroxyhexansäure, Tetrahydroxypentansäure und/oder deren Derivate, wie z.B. Ether, Ester und/oder Amide, z.B. Pentahydroxyhexansäureamid und/oder deren physiologisch verträglichen Salzen;_weitere Beispiele: Zitronensäure, Äpfelsäure oder Weinsäure;
- Pantolacton;
- Panthenol und/oder dessen Derivate;
- weitere Vitamine, wie z.B. Vitamin B6, C und/oder E und/oder deren Derivate;
- Hydroxysäuren, wie z.B. α-, β-Hydroxyfettsäuren bzw. Ketofettsäuren und/oder deren physiologisch verträglichen Salzen; wie beispielsweise Salicylsäure oder Milchsäure, Glyoxylsäure, Glycolsäure.
- wasserlösliche Polymere festigender Wirkung, z.B. Polyvinylpyrrolidon, Vinylacetat/Crotonsäure-Copolymere,
- Antischuppenwirkstoffe wie z.B. Picrotone Olamine, Zink Omadine,
- Wirkstoffe wie Allantoin, Pyrrolidoncarbonsäuren, Pflanzenextrakte ,
- pH-Einstell- und Puffermittel wie z.B. Citronensäure/Natriumcitrat, Ammoniumcarbonat, Ammoniumhydrogencarbonat, Guanidincarbonat, Ammoniak, Natriumhydroxyd,
- Komplexbildner wie EDTA, NTA, Organophosphonsäuren, Dipicolinsäure, Salicylsäure,
- Lichtschutzmittel (UV-Absorber),
- Öl-, Fett- und Wachskomponenten, bevorzugt in emulgierter Form,
- Farbstoffe, Trübungs- und Perlglanzmittel sowie
- gegebenenfalls Aerosol-Treibgase.

Im Anschluss an die Durchführung des erfindungsgemäßen Verfahrens können die keratinischen Fasern in üblicher Weise nachbehandelt werden. Beispielsweise kann die Anwendung eines handelsüblichen Conditioner vorteilhaft sein. Eine Behandlung mit einem Conditioner kann auch zwischen Schritt (iii) und (iv) des erfindungsgemäßen Verfahrens erfolgen.

Die Erfindung wird anhand der folgenden Beispiele weiter erläutert, wobei die Beispiele das Verständnis des erfindungsgemäßen Prinzips erleichtern sollen und nicht als Einschränkung zu verstehen sind.

### Beispiele

Es wurde ein Umformungsmittel, d.h. eine wässrige, keratinreduzierende Zusammensetzung, gemäß Tabelle 1 und ein Färbemittel gemäß Tabelle 2 nach einem Standardverfahren hergestellt. Ferner wurden nach bekannter Vorgehensweise zwei Fixiermittel, d.h. oxidierende Zusammensetzungen, gemäß Tabelle 3 bereitgestellt.

**Tabelle 1: Umformungsmittel**

| Rohstoff | [Gew.%] |
|---|---|
| Cremophor^{®} CO 40 ¹ | 1,00 |
| Lamepon^{®} S ² | 1,00 |
| Natrosol^{®} 250 HR ³ | 1,25 |
| Ammoniak (25%ige wässrige Lösung) | 0,95 |
| Turpinal^{®} SL ⁴ | 0,30 |
| Ammoniumthioglykolat (71 %ige wässrige Lösung) | 16,70 |
| Ammoniumbicarbonat | 7,05 |
| Parfüm | 0,50 |
| Wasser | ad 100 |

| | |
|---|---|
| ¹ hydriertes Rizinusöl mit ca. 40-45 EO-Einheiten (INCI-Bezeichnung: PEG-40 Hydrogenated Castor Oil) (BASF) ² Kaliumsalz eines Kollagen-Kokosfettsäurekondensats, 31,5-32,5% Trockensubstanz (INCI-Bezeichnung: Aqua (Water), Potassium Cocoyl Hydrolyzed Collagen, Phenoxyethanol, Methylparaben, Ethylparaben) (Cognis) ³ Hydroxyethylcellulose (Hercules) ⁴ 1-Hydroxyethan-1,1-diphosphonsäure (INCI-Bezeichnung: Etidronic Acid, Aqua (Water)) (Solutia) | |

**Tabelle 2: Färbemittel**

| Rohstoff | [Gew.%] |
|---|---|
| Carbopol^{®} 934 ⁵ | 1,30 |
| KOH (50%ige wässrige Lösung) | 1,90 |
| Emulgade^{®} F ⁶ | 3,00 |
| Dehyton^{®} G ⁷ | 5,00 |
| DL-Serin | 1,00 |
| Natriumsulfit | 0,50 |
| Kieselsäure, hochdispers | 0,25 |
| p-Toluylendiaminsulfat | 4,40 |
| Resorcin | 1,10 |
| 3-Aminophenol | 0,66 |
| 1,3-Bis-(2,4-diaminophenoxy)-propan | 1,74 |
| Parfüm | 0,10 |
| Wasser | ad 100 |

| | |
|---|---|
| ⁵ Polyarcylsäure quervernetzt mit Polyalkenylpolyether (INCI-Bezeichnung: Carbomer) (Noveon) ⁶ Mischung aus Cetylstearylalkohol, Rizinusöl mit ca. 40-EO-Einheiten und dem Natriumsalz von Cetylstearylsulfat (INCI-Bezeichnung: Cetearyl Alcohol, PEG-40 Castor Oil, Sodium Cetearyl Sulfate) (Cognis) ⁷ N-(2-Hydroxyethyl)-N-(kokosamidoethyl)carboxymethylglycinat-Natrium-Salz (ca. 39-31% Aktivsubstanzgehalt; INCI-Bezeichnung: Aqua (Water), Disodium Cocoamphodiacetate) (Cognis) | |

**Tabelle 3: Fixiermittel**

| | Fixiermittel Nr. | |
|---|---|---|
| Rohstoff | F1 [Gew.%] | F2 [Gew.%] |
| Eumulgin^{®} L ⁸ | 0,30 | - |
| Ammoniak (25 %ige wässrige Lösung) | 0,50 | - |
| Dipicolinsäure | 0,10 | - |
| Aromox^{®} MCD-W ⁹ | 3,00 | 3,00 |
| Turpinal^{®} SL ⁴ | 1,60 | - |
| Dehyquart^{®} A-CA ¹⁰ | 0,18 | 0,20 |
| Merquat^{®} 100 ¹¹ | 0,30 | 0,25 |
| Cremophor^{®} CO 40 ¹ | 0,30 | - |
| Methylparaben | - | 0,04 |
| Ortho-Phosphorsäure | - | 0,95 |
| Parfüm | 0,30 | 0,30 |
| Wasserstoffperoxid (50 %ige wässrige Lösung) | 12,00 | 4,00 |
| Wasser | ad 100 | ad 100 |

| | | |
|---|---|---|
| ⁸ Laurylglykolether, ethoxyliert mit 1 Einheit Propylenoxid und 9 Einheiten Ethylenoxid (INCI-Bezeichnung: PPG-1-PEG-9 Lauryl Glycol Ether) (Cognis) ⁹ N,N-Dimethyl-N-kokosalkylamin-N-oxid (ca. 30% Aktivsubstanz; INCI-Bezeichnung: Cocamine Oxide) (Akzo Nobel) ¹⁰ Trimethylhexadecylammoniumchlorid (ca. 24 -26 % Aktivsubstanz; INCI-Bezeichnung: Aqua (Water), Cetrimonium Chloride) (Cognis) ¹¹ Poly(dimethyldiallylammoniumchlorid) (ca. 40% Festkörper; INCI-Bezeichnung: Polyquaternium-6) (Ondeo Nalco) | | |

### Versuchsdurchführung und Beurteilung der Ergebnisse:

Ungeschädigtes Haar wurde mit Wasser angefeuchtet und mit einem Handtuch frottiert.

Die Glättungscreme und das Färbemittel wurde im Gewichtsverhältnis von 1 : 1 vermischt und unmittelbar nach dem Mischen gleichmäßig auf das gesamte Haar aufgebracht. Nach einer Einwirkungszeit von 20 Minuten wurde das Haar gespült, vorsichtig mit einem Handtuch frottiert und auf Wickler mit einem Durchmesser von 8 Millimeter gewickelt. Nach etwa 10 Minuten wurde das Haar sodann mit einem der Fixiermittel gemäß Tabelle 1 behandelt. Nach einer Einwirkungszeit von 15 Minuten wurden die Wickler entfernt, das Haar mit Wasser gespült und schließlich getrocknet. Dieses Verfahren wurde mit beiden Fixiermitteln F1 bis F2 durchlaufen.

Unabhängig vom eingesetzten Fixiermittel wurde jeweils ein sehr gleichmäßiges Well- und Färbeergebnis erzielt.

## Patentansprüche

1. Verfahren zum gleichzeitigen Färben und dauerhaften Verformen keratinhaltiger Fasern, insbesondere menschlicher Haare, **dadurch gekennzeichnet, dass**
(i) eine wässrige, keratinreduzierende Zusammensetzung, enthaltend mindestens eine keratinreduzierende Verbindung und mindestens ein Oxidationsfarbstoffvorprodukt und/oder mindestens einen direktziehenden Farbstoff, auf die Fasern aufgetragen wird,
(ii) die Fasern nach einer Einwirkzeit Z1 gespült und gegebenenfalls getrocknet werden,
(iii) die Fasern anschließend unter Zuhilfenahme von Verformungshilfsmitteln verformt werden und
(iv) schließlich eine oxidierende Zusammensetzung, enthaltend mindestens eine oxidierende Verbindung, auf die Fasern aufgetragen und nach einer Einwirkzeit Z2 wieder abgespült wird.

2. Verfahren nach Anspruch 1, **dadurch gekennzeichnet, dass** die keratinhaltigen Fasern vor dem Schritt (i) angefeuchtet werden.

3. Verfahren nach einem der Ansprüche 1 oder 2, **dadurch gekennzeichnet, dass** die keratinreduzierende Verbindung ausgewählt ist aus Thioglykolsäure, Thiomilchsäure, Thioäpfelsäure, Phenylthioglykolsäure, Mercaptoethansulfonsäure sowie deren Salzen und Estern, Cysteamin, Cystein, Bunte-Salzen und Salzen der schwefligen Säure, Alkalidisulfiten, wie z.B. Natriumdisulfit (Na₂S₂O₅) und Kaliumdisulfit (K₂S₂O₅), sowie Magnesiumdisulfit und Ammoniumdisulfit ((NH₄)₂S₂O₅), Hydrogensulfiten als Alkali-, Magnesium-, Ammonium- oder Alkanolammonium-Salze auf Basis eines C₂-C₄-Mono-, Di- oder Trialkanolamins, sowie Sulfiten als Alkali-, Ammonium- oder Alkanolammonium-Salze auf Basis eines C₂-C₄-Mono-, Di- oder Trialkanolamins.

4. Verfahren nach Anspruch 3, **dadurch gekennzeichnet, dass** die keratinreduzierende Verbindung ausgewählt ist aus Thioglykolsäure, Thiomilchsäure und Cystein sowie deren Salzen.

5. Verfahren nach einem der Ansprüche 1 bis 4, **dadurch gekennzeichnet, dass** die keratinreduzierenden Verbindungen in einer Menge von 1-25 Gew.-%, bevorzugt 5-15 Gew.-%, bezogen auf die gesamte wässrige, keratinreduzierende Zusammensetzung, enthalten sind.

6. Verfahren nach einem der Ansprüche 1 bis 5, **dadurch gekennzeichnet, dass** in der wässrigen, keratinreduzierenden Zusammensetzung zusätzlich mindestens ein anionisches, nichtionisches, amphoteres oder zwitterionisches Tensid enthalten ist.

7. Verfahren nach einem der Ansprüche 1 bis 6, **dadurch gekennzeichnet, dass** die Oxidationsfarbstoffvorprodukte und/oder die direktziehenden Farbstoffe in einer Menge von 0,1 bis 20 Gew.-%, bezogen auf die gesamte wässrige, keratinreduzierende Zusammensetzung, enthalten sind.

8. Verfahren nach einem der Ansprüche 1 bis 7, **dadurch gekennzeichnet, dass** als oxidierende Verbindung Natriumbromat, Kaliumbromat oder Wasserstoffperoxid eingesetzt wird.

9. Verfahren nach einem der Ansprüche 1 bis 8, **dadurch gekennzeichnet, dass** als oxidierende Verbindung Wasserstoffperoxid eingesetzt wird.

10. Verfahren nach Anspruch 9, **dadurch gekennzeichnet, dass** Wasserstoffperoxid in einer Menge von 0,5 bis 3,0 Gew.-%, bezogen auf die gesamte oxidierende Zusammensetzung, enthalten ist.

11. Verfahren nach einem der Ansprüche 1 bis 10, **dadurch gekennzeichnet, dass** die keratinischen Fasern zwischen den Behandlungsschritten (ii) und (iii) keinerlei weitere Behandlung erfahren.
